# EUROPEAN PATENT SPECIFICATION

(11) **EP 1 557 473 B1**
(45) Date of publication and mention of the grant of the patent: **11.03.2009**
(21) Application number: 03769947.7
(22) Date of filing: 29.10.2003
(51) Int. Cl.: C12Q 1/34

(54) **REAGENT COMPOSITION FOR DISCRIMINATING BETA-LACTAMASES, KIT AND METHOD THEREOF**
REAGENZZUSAMMENSETZUNG, KIT UND VERFAHREN ZUR UNTERSCHEIDUNG VON BETA-LACTAMASEN
COMPOSITION REACTIVE POUR DISCRIMINER ENTRE DIFFERENTES CLASSES DE BETA-LACTAMASES, KIT ET METHODE ASSOCIES

(30) Priority: 29.10.2002 JP 2002314681
(43) Date of publication of application: 27.07.2005
(73) Proprietor: Showa Yakuhin Kako Co., Ltd, Tokyo 104-0031 (JP); Hanaki, Hideaki, Hiratsuka-shi Kanagawa-ken 259-1217 (JP)
(72) Inventor: MURATA, Atsushi, Kawasaki-shi, Kanagawa 213-0006 (JP)
(74) Representative: Zimmermann & Partner
(86) International application number: PCT/JP2003/013835
(87) International publication number: WO 2004/040008

(56) References cited:
- WO-A-02/24707
- WO-A1-02/24707
- WO-A1-92/19763
- WO-A1-96/21039
- WO-A2-02/74287
- WO-A2-96/30540
- JP-A- 2000 224 998
- US-A- 3 830 700
- US-A- 5 061 702
- APPELBAUM P C ET AL: "CHARACTERIZATION OF A BETA-LACTAMASE FROM CLOSTRIDIUM CLOSTRIDIOFORME" JOURNAL OF ANTIMICROBIAL CHEMOTHERAPY, SAUNDERS CO. LTD., LONDON, GB, vol. 33, no. 1, 1994, pages 33-40, XP008033659 ISSN: 0305-7453
- CROSBY M A ET AL: "Activity of Cefoperazone and two b-Lactamase Inhibitors, Sulbactam and Clavulanic Acid, Against Bacteroides spp. correlated with b-Lactamase Production" ANTIMICROBIAL AGENTS AND CHEMOTHERAPY, AMERICAN SOCIETY FOR MICROBIOLOGY, WASHINGTON, DC, US, vol. 22, no. 3, 1982, pages 398-405, XP002977703 ISSN: 0066-4804
- HURLBUT S. ET AL: 'Imipenem Resistance in Bacteroides distasonis Mediated by a Novel b-Lactamase' ANTIMICROBIAL AGENTS AND CHEMOTHERAPY vol. 34, no. 1, 1990, pages 117 - 120, XP002977701
- P. C. APPELBAUM ET AL: 'Characterization of a b-Lactamase from Clostridium clostridioforme' JOURNAL OF ANTIMICROBIAL CHEMOTHERAPY vol. 33, 1994, pages 33 - 40, XP008033659
- M. A. CROSBY ET AL: 'Activity of Cefoperazone and two b-Lactamase Inhibitors, Sulbactam and Clavulanic Acid, Against Bacteroides spp. correlated with b-Lactamase Production' ANTIMICROBIAL AGENTS AND CHEMOTHERAPY vol. 22, no. 3, 1982, pages 398 - 405, XP002977703
- BUSH K. ET AL: 'A functional classification scheme for beta-lactamases and its correlation with molecular structure' ANTIMICROBIAL AGENTS AND CHEMOTHERAPY vol. 39, no. 6, 01 June 1995, UNITED STATES, pages 1211 - 1233
- GALLENI M. ET AL: 'A survey of the kinetic parameters of class C beta-lactamases. Cephalosporins and other beta-lactam compounds' THE BIOCHEMICAL JOURNAL vol. 255, no. 1, 01 October 1988, ENGLAND, pages 123 - 129
- YAMAGUCHI T.; KOHNO S.: 'Clinical characteristics of emerging multiple-drug-resistant gram-negative rods producing extended-spectrum beta-lactamases (ESBLs)' NIPPON RINSHO. JAPANESE JOURNAL OF CLINICAL MEDICINE, [Online] vol. 59, no. 4, 01 April 2001, JAPAN, pages 750 - 755 Retrieved from the Internet: <URL:http://www.ncbi.nlm.nih.gov/pubmed/113 05001?dopt=Abstract>

## Description

### Technical Field

The present invention relates to a reagent composition for discriminating β -lactamase, a kit for detecting β -lactamase and a β -lactamase discrimination method.

### Background Art

β -lactamases, which are a family of enzymes that hydrolyze the β -lactam ring contained in a molecule of β -lactam based antibacterial agents, are classified into class A, B, C and D according to amino-acid sequence homology in the enzyme proteins. Extended-spectrum β -lactamase (ESBL) is an enzyme that has a more extended range of substrates (i.e., β -lactam based antibacterial agents) to be decomposed by the enzyme as compared with the conventional class A β -lactamase. These β -lactamases deactivate the antibacterial action of the β **-**lactam based antibacterial agents. Particularly, the ESBL has been recognized as a cause of nosocomial infection and perceived as a serious problem. Administration of β -lactam based antibacterial agents against the bacteria having developed a resistance to the β -lactam based antibacterial agents not only would be a hopeless cure, but also might lead to spreading of new resistant bacteria. Thus, it is necessary to detect the β -lactamases rapidly and accurately in determining a sure cure.

The methods currently used for detecting the β -lactamases are roughly divided into the following four methods: (1) chromogenic cephalosporin method, (2) acidmetry method, (3) iodometry method, and (4) UV method. In addition, there can be employed a cultivation method for comparing the minimum inhibitory concentrations (MIC). Principal among the commercially available products for detecting the β -lactamases are: a product capable of indicating whether β -lactamase is present or absent using the chromogenic cephalosporin method; a product capable of detecting the β -lactamases belonging to the class A and class C using the acidmetry method; a product capable of detecting the class B β -lactamase or ESBL using the cultivation method, and the like.

The chromogenic cephalosporin method uses cephalosporin that will cause a color change upon the cleavage of its β -lactam ring by the application of β -lactamase thereto. This method has the advantage that the detection sensitivity is excellent because the reagent itself results in a color change. However, the conventional commercially available products using the chromogenic cephalosporin method employ as a detection substrate nitrocefin (i.e., 3-[2,4-dinitrostyryl]-7-(2-thienylacetamido)-3-cephem-4-carboxylic acid in Japanese Patent Unexamined Publication (JP Kokai) Sho 48-50787). This product cannot react to all the classes of β -lactamases and cannot distinguish one class from the others, although the detection can be achieved in a short period of time, i.e., about 30 minutes. Further, among the products based on the chromogenic cephalosporin method, no product is conventionally known that is characterized by using nitrocefin in combination with a β -lactamase inhibitor.

A novel compound capable of detecting the ESBL based on the chromogenic cephalosporin method is disclosed in PCT pamphlet WO 02/24707. However, this PCT pamphlet does not disclose the combined use of the compound and a particular β -lactamase inhibitor, nor suggest the possibility of detecting any classes of β -lactamases in addition to the ESBL.

### Disclosure of Invention

An object of the present invention is to provide a reagent composition for discriminating β -lactamase, a kit for discriminating β -lactamase and a β -lactamase discrimination method, which allow rapid discrimination of the β -lactamases.

The inventors of the present invention have found that the above-mentioned object can be achieved by the combined use of a particular β -lactamase detection substrate and a β -lactamase inhibitor, wherein the β -lactamase inhibitor is selected from the group consisting of a combination of aztreonam and ethylendiaminetetraacetic acid; a combination of clavulanic acid and ethylendiaminetetraacetic acid; a combination of aztreonam and clavulanic acid.

Namely, the present invention provides a reagent composition for discriminating β -lactamase for use with a chromogenic cephalosporin method, comprising a β -lactamase detection substrate and a β -lactamase inhibitor.

The present invention also provides a kit for discriminating β -lactamase comprising one or more detecting reagent compositions selected from the group consisting of (a) to (d) shown below:
(a) a detecting reagent composition comprising a compound represented by general formula (1) or physiologically acceptable salt thereof as a β -lactamase detection substrate, and
   a combination of aztreonam and ethylenediaminetetraacetic acid as a β -lactamase inhibitor;
(b) a detecting reagent composition comprising a compound represented by general formula (1) or physiologically acceptable salt thereof as a β -lactamase detection substrate, and
   a combination of clavulanic acid and ethylenediaminetetraacetic acid as a β -lactamase inhibitor;
(c) a detecting reagent composition comprising a compound represented by general formula (1) or physiologically acceptable salt thereof as a β -lactamase detection substrate, and
   a combination of aztreonam and clavulanic acid as a β -lactamase inhibitor;
(d) a detecting reagent composition comprising a compound represented by general formula (2) or carboxylate derivative thereof as a β -lactamase detection substrate, and
   a combination of aztreonam and ethylenediaminetetraacetic acid as a β -lactamase inhibitor.
   The present invention also provides a kit for discriminating β -lactamase comprising one or more detecting reagent compositions selected from the group consisting of (f), (g), (h) and (i) shown below:
(f) a detecting reagent composition comprising a compound represented by general formula (1) or physiologically acceptable salt thereof as a β -lactamase detection substrate, and
   aztreonam as a β -lactamase inhibitor;
(g) a detecting reagent composition comprising a compound represented by general formula (1) or physiologically acceptable salt thereof as a β -lactamase detection substrate, and
   clavulanic acid as a β -lactamase inhibitor;
(h) a detecting reagent composition comprising a compound represented by general formula (2) or carboxylate derivative thereof as a β -lactamase detection substrate, and
   aztreonam as a β -lactamase inhibitor; and
(i) a detecting reagent composition comprising a compound represented by general formula (2) or carboxylate derivative thereof as a β -lactamase detection substrate, and
   a combination of aztreonam and clavulanic acid as a β -lactamase inhibitor.

In addition, the present invention provides a β -lactamase detection method comprising the step of bringing a liquid specimen containing a target substance to be analyzed in contact with the above-mentioned reagent composition for detecting β -lactamase.

Further, the present invention provides a β -lactamase discrimination method, comprising the step of bringing a liquid specimen containing a target substance to be analyzed into contact with the above-mentioned reagent composition for detecting β -lactamase held in a sample holding portion of a kit for detecting β -lactamase .

The present invention also provides a β -lactamase discrimination method, comprising the step of bringing a liquid specimen containing a target substance to be analyzed into contact with a reagent composition for detecting β -lactamase held in a sample holding portion of the above-mentioned kit for detecting β -lactamase

### Brief Description of Drawing

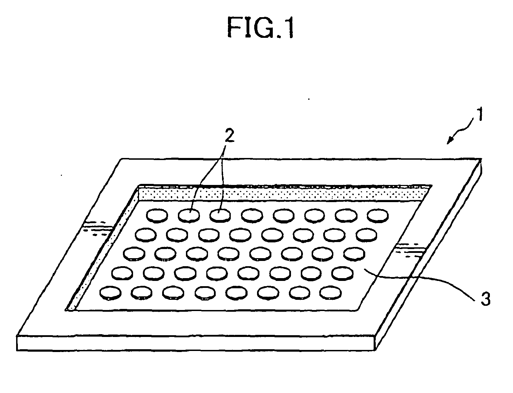

Fig. 1 is a schematic perspective view showing one embodiment of the kit for discriminating β -lactamase according to the present invention.

### Best Mode for Carrying out the Invention

The present invention is based on the chromogenic cephalosporin method.

A compound represented by the following general formula (1) or physiologically acceptable salts thereof disclosed in JP Kokai Sho 48-50787, or a compound represented by the following general formula (2) or carboxylate derivatives thereof disclosed in WO 02/24707, which patent literatures are incorporated herein in their entirety by reference, may be used as the substrate for detecting the β-lactamases for use in the present invention.

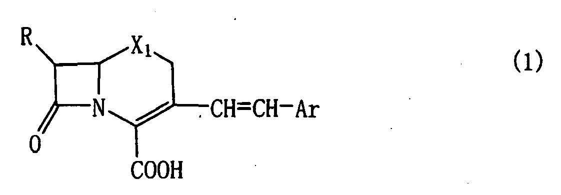

In general formula (1), R is a formamide group or a group represented by formula of R^{u}CH₂CONH, R^{u}OCH₂CONH, R^{u}CONH, R^{u}CH(NH₂)CONH or R^{u}C(=NOH)CONH, wherein R^{u} is thienyl group, phenyl group or naphthyl group; Ar is a phenyl group having a substitution of cyano group or nitro group at the 2-, 4-, or 2,4-position; and -X₁- is -S- or -SO-.

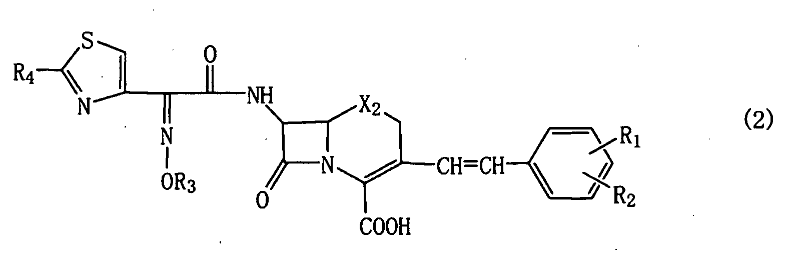

In general formula (2), R₁ and R₂, which may be the same or different, are each hydrogen atom, nitro group, or cyano group; R₃ is an alkyl group having 1 to 6 carbon atoms which may have a substituent of carboxyl group; R₄ is hydrogen atom or amino group; and -X₂- is -S- or -SO-, provided that R₁ and R₂ do not represent hydrogen atom at the same time.

The compound represented by general formula (1), serving as the β -lactamase detection substrate may preferably be 3-[2,4-dinitrostyryl]-7-(2-thienylacetamido)-3-cephem-4-carboxylic acid or physiologically acceptable salts thereof.

Preferable compounds represented by general formula (2) include a compound wherein -X₂- is -S-; a compound wherein R₃ is methyl group which may be substituted with carboxyl group or propyl group substituted with carboxyl group; a compound wherein R₄ is amino group; and a compound wherein -X₂- is -S-, R₃ is methyl group which may be substituted with carboxyl group or propyl group substituted with carboxyl group, and R₄ is amino group.

The following compounds and carboxylate derivatives thereof are preferable:
7-[2-(2-aminothiazol-4-yl)-2-(1-carboxy-1-methylethoxy-imino)acetamido]-3-(2,4-dinitrostyryl)-3-cephem-4-carboxylic acid,
7-[2-(2-aminothiazol-4-yl)-2-(1-carboxy-1-methylethoxy-imino)acetamido]-3-(2,6-dinitrostyryl)-3-cephem-4-carboxylic acid,
7-[2-(2-aminothiazol-4-yl)-2-(1-carboxy-1-methylethoxy-imino)acetamido]-3-(4-nitrostyryl)-3-cephem-4-carboxylic acid,
7-[2-(2-aminothiazol-4-yl)-2-(1-carboxy-1-methylethoxy-imino)acetamido]-3-(2,4-dicyanostyryl)-3-cephem-4-carboxylic acid,
7-[2-(2-aminothiazol-4-yl)-2-(1-carboxy-1-methylethoxy-imino)acetamido]-3-(4-cyanostyryl)-3-cephem-4-carboxylic acid,
7-[2-(2-aminothiazol-4-yl)-2-(1-carboxy-1-methylethoxy-imino)acetamido]-3-(2-cyanostyryl)-3-cephem-4-carboxylic acid,
7-[2-(1-carboxy-1-methylethoxyimino)-2-(thiazol-4-yl)acetamido]-3-(2,4-dinitrostyryl)-3-cephem-4-carboxylic acid,
7-[2-(2-aminothiazol-4-yl)-2-(1-carboxy-1-methylethoxy-imino)acetamido]-3-(2,4-dinitrostyryl)-3-cephem-4-carboxylic acid-1-oxide,
7-[2-(2-aminothiazol-4-yl)-2-methoxyiminoacetamido]-3-(4-nitrostyryl)-3-cephem-4-carboxylic acid,
7-[2-(2-aminothiazol-4-yl)-2-methoxyiminoacetamido]-3-(2,4-dicyanostyryl)-3-cephem-4-carboxylic acid,
7-[2-(2-aminothiazol-4-yl)-2-methoxyiminoacetamido]-3-(2,6-dicyanostyryl)-3-cephem-4-carboxylic acid,
7-[2-(2-aminothiazol-4-yl)-2-methoxyiminoacetamido]-3-(2-cyanostyryl)-3-cephem-4-carboxylic acid,
7-[2-(2-aminothiazol-4-yl)-2-carboxymethoxyimino-acetamido]-3-(2,4-dinitrostyryl) -3-cephem-4-carboxylic acid,
7-[2-(2-aminothiazol-4-yl)-2-carboxymethoxyimino-acetamido]-3-(2,6-dinitrostyryl) -3-cephem-4-carboxylic acid,
7-[2-(2-aminothiazol-4-yl)-2-carboxymethoxyimino-acetamido]-3-(4-nitrostyryl)-3-cephem-4-carboxylic acid,
7-[2-(2-aminothiazol-4-yl)-2-carboxymethoxyimino-acetamido]-3-(2-nitrostyryl)-3-cephem-4-carboxylic acid,
7-[2-(2-aminothiazol-4-yl)-2-carboxymethoxyimino-acetamido]-3-(2,4-dicyanostyryl)-3-cephem-4-carboxylic acid,
7-[2-(2-aminothiazol-4-yl)-2-carboxymethoxyimino-acetamido]-4-(4-cyanostyryl)-3-cephem-4-carboxylic acid,
7-[2-(2-aminothiazol-4-yl)-2-carboxymethoxyimino-acetamido]-4-(2-cyanostyryl)-3-cephem-4-carboxylic acid,
7-[2-carboxymethoxyimino-2-(thiazol-4-yl)acetamido]-3-(2,4-dinitrostyryl)-3-cephem-4-carboxylic acid, and
7-[2-(2-aminothiazol-4-yl)-2-carboxymethoxyimino-acetamido]-3-(2,4-dinitrostyryl) -3-cephem-4-carboxylic acid-1-oxide.

In particular, the following compounds and carboxylate derivatives thereof are preferable:
7-[2-(2-aminothiazol-4-yl)-2-(1-carboxy-1-methylethoxy-imino)acetamido]-3-(2,4-dinitrostyryl)-3-cephem-4-carboxylic acid,
7-[2-(2-aminothiazol-4-yl)-2-(1-carboxy-1-methylethoxy-imino)acetamido]-3-(4-nitrostyryl)-3-cephem-4-carboxylic acid,
7-[2-(2-aminothiazol-4-yl)-2-(1-carboxy-1-methylethoxy-imino)acetamido]-3-(4-cyanostyryl)-3-cephem-4-carboxylic acid,
7-[2-(2-aminothiazol-4-yl)-2-methoxyiminoacetamido]-3-(4-nitrostyryl)-3-cephem-4-carboxylic acid,
7-[2-(2-aminothiazol-4-yl)-2-carboxymethoxyimino-acetamido]-3-(2,4-dinitrostyryl) -3-cephem-4-carboxylic acid, and
7-[2-(2-aminothiazol-4-yl)-2-carboxymethoxyimino-acetamido]-3-(4-nitrostyryl)-3-cephem-4-carboxylic acid.

In particular, 7-[2-(2-aminothiazol-4-yl)-2-(1-carboxy-1-methylethoxyimino)acetamido]-3-(2,4-dinitrostyryl)-3-cephem-4-carboxylic acid or 7-[2-(2-aminothiazol-4-yl)-2-carboxymethoxyiminoacetamido]-3-(2,4-dinitrostyryl)-3-cephem-4-carboxylic acid is preferable.

As the β -lactamase inhibitor, at least one selected from the group consisting of clavulanic acid or aztreonam may be used. The chelating agent - which is capable of chelation of zinc ion - is ethylenediaminetetraacetic acid (EDTA).

In particular, it is preferable that the β -lactamase inhibitor be selected from the group consisting of clavulanic acid; aztreonam; a combination of aztreonam and ethylenediaminetetraacetic acid; a combination of clavulanic acid and ethylenediaminetetraacetic acid; a combination of aztreonam and clavulanic acid.

Examples of the combined use of the β -lactamase detection substrate and the β -lactamase inhibitor, preferably employed in the present invention are as follows:
a detecting reagent composition comprising a compound represented by the above-mentioned general formula (1) or physiologically acceptable salt thereof as the β -lactamase detection substrate, and at least one β -lactamase inhibitor selected from the group consisting of clavulanic acid, aztreonam, and ethylenediaminetetraacetic acid; and
a detecting reagent composition comprising a compound represented by the above-mentioned general formula (2) or carboxylate derivative thereof as the β -lactamase detection substrate, and at least one β -lactamase inhibitor selected from the group consisting of clavulanic acid, aztreonam, and ethylenediaminetetraacetic acid.

In addition, a detecting reagent composition including the above-mentioned composition (c), and further one or more compositions selected from the group consisting of (f) through (i) shown below is also preferable:
(f) a detecting reagent composition comprising a compound represented by general formula (1) or physiologically acceptable salt thereof as the β -lactamase detection substrate, and aztreonam as the β -lactamase inhibitor;
(g) a detecting reagent composition comprising a compound represented by general formula (1) or physiologically acceptable salt thereof as the β -lactamase detection substrate, and clavulanic acid as the β -lactamase inhibitor;
(h) a detecting reagent composition comprising a compound represented by general formula (2) or carboxylate derivative thereof as the β -lactamase detection substrate, and aztreonam as the β -lactamase inhibitor; and
(i) a detecting reagent composition comprising a compound represented by general formula (2) or carboxylate derivative thereof as the β -lactamase detection substrate, and a combination of aztreonam and clavulanic acid as the β -lactamase inhibitor.

In the detecting reagent composition of the present invention, the ratio by mass of the β -lactamase detection substrate to the β -lactamase inhibitor may preferably be in the range of (1:10) to (100:1), more preferably in the range of (1:1) to (50:1), and further preferably in the range of (1:1) to (1:10).

The detecting reagent composition of the present invention may be prepared in the form of a solution by dissolving the β -lactamase detection substrate and the β -lactamase inhibitor in a solvent, such as water, phosphate buffer solution, dimethyl sulfoxide, or a mixture thereof. Of those solvents, a mixture of dimethyl sulfoxide and phosphate buffer solution is especially preferable, and in this case, the mixing ratio is not particularly limited. To be more specific, the β-lactamase detection substrate and the β -lactamase inhibitor may be dissolved in a solvent so that the concentration of the β -lactamase detection substrate may preferably be in the range of 0.025 to 62.5 mg/mL, more preferably 1.25 to 12.5 mg/mL, and that of the β -lactamase inhibitor may preferably be in the range of 0.0125 to 12.5 mg/mL, more preferably 1.25 to 12.5 mg/mL, thereby obtaining a solution containing a reagent composition for detecting β -lactamase.

It is preferable to use the detecting reagent composition of the present invention adjusted to have pH 6.0 to 8.0. The detecting reagent composition may preferably be adjusted to about pH 5.5 to 8.5 in the case where the compound represented by the aforementioned general formula (1) is used as the β -lactamase detection substrate. When the compound represented by the aforementioned general formula (2) is used as the β -lactamase detection substrate, the detecting reagent composition may preferably be adjusted to about pH 6.0 to 8.0.

The solution containing detecting reagent composition thus prepared may further comprise polymers or the like, such as polyvinylpyrrolidone, hydroxymethyl cellulose, polyvinyl chloride, and methacrylic acid copolymer in such an amount that may not hinder the effects of the present invention. Addition of such polymers or the like is advantageous because the solution containing the detecting reagent composition of the present invention becomes more stable. Of those polymers particularly preferable is methacrylic acid copolymer.

The specimen used in the present invention can be collected, for example, from pharynx and nasal cavity of patients with a cotton swab, and in addition, from urine, sputum, pus and the like. The specimen may be prepared in a liquid form by dissolving the specimen in a solvent such as water, phosphate buffer solution, physiological saline solution or the like. Preferably, the liquid specimen may be prepared to have a bacteria concentration of 10⁴ to 10¹⁰ CFU/mL.

The liquid specimen thus prepared may further contain oligosaccharide, surfactant, dextrin, and the like in such amounts that may not impair the effects of the present invention.

The kit for discriminating β -lactamase of the present invention may be in any form so long as the above-mentioned detecting reagent composition is contained therein, for example, in the form of a disk, combination of a disk and a board, a plate, a strip or the like. The form preferably used is a disk-type one.

When necessary, the kit of the present invention may include a positive control liquid made of a solution containing β -lactamase and the detecting reagent composition of the present invention, and a negative control liquid made of a solution containing the β -lactamase detection substrate.

Fig. 1 is a schematic perspective view showing a kit for discriminating β -lactamases according to the present invention, but the present invention is not limited thereto. Referring to Fig. 1, a discrimination kit according to the present invention includes a sample container 1, and a base member 3 which is stored in the container and bears a plurality of sample holding portions 2 thereon. The sample holding portion 2 has an opening on which a prepared sample (a liquid specimen or control liquid) is dropped. The materials for the sample container, base member, and the sample holding portion, which constitute the detection kit of the present invention, are not particularly limited. There is no restraint on the size and the shape of such parts.

One of the preferable kits according to the present invention is the one that contains the above-mentioned detecting reagent composition (c) as an essential composition, and further contains one or more detecting reagent compositions selected from the group consisting of the compositions (a), (b) and (d).

Also, the kit for detecting β -lactamase that contains the detecting reagent composition (d) as an essential composition, and further contains one or more detecting reagent compositions selected from the group consisting of the compositions (a), (b) and (c) is preferred.

In particular, the kit for discriminating β -lactamase that contains all the above-mentioned detecting reagent compositions (a) through (d) is preferable.

In addition, preferably employed is the kit according to the present invention that contains one or more detecting reagent compositions selected from the group consisting of the compositions (c) and (f) through (i).

More preferably used is the kit according to the present invention that contains the above-mentioned detecting reagent composition (c) as an essential composition, and further contains one or more detecting reagent compositions selected from the group consisting of the compositions (f), (g), (h) and (i).

Also, preferably used is the kit for discriminating β -lactamase according to the present invention that contains the above-mentioned detecting reagent composition (h) as an essential composition, and further contains one or more detecting reagent compositions selected from the group consisting of the compositions (c), (f), (g) and (i).

In particular, the kit for detecting β -lactamase that contains all the above-mentioned detecting reagent compositions (c) and (f) through (i) is further preferable.

In the discrimination kit of the present invention, it is most preferable that 3-[2,4-dinitrostyryl]-7-(2-thienylacetamido]-3-cephem-4-carboxylic acid or 7-[2-(2-aminothiazol-4-yl)-2-(1-carboxy-1-methylethoxy-imino)acetamido]-3-(2,4-dinitrostyryl)-3-cephem-4-carboxylic acid (hereinafter referred to as "HMRZ compound") be contained as the substrate for detecting the β-lactamases in the above-mentioned detecting reagent compositions (a) to (d) and (f) to (i).

By using the kit according to the present invention, the class A, B, C and D β -lactamases, and ESBL can be discriminated rapidly by one operation. Therefore, use of the kit according to the present invention will lead to choice of a proper antibacterial agent, which can further enhance the effects of cure.

The present invention also relates to a β -lactamases discrimination method, comprising the step of adding a liquid specimen containing a target substance to be analyzed to a solution containing any of the above-mentioned reagent composition for discriminating β -lactamases.

The method of the present invention includes the step of adding a liquid specimen containing a target substance to be analyzed to the solution containing the reagent composition for detecting β -lactamase prepared in such a manner as described above. It is possible to discriminate between positive and negative by checking whether the color tone is changed from yellow to red, or not, after a lapse of a given time, for example, about 30 minutes, at a predetermined temperature, for example, at room temperature. Further, the activity assay can also be performed by measuring a change in color tone at a particular wavelength.

By using the method of the present invention, it is possible to rapidly detect β -lactamases in urine and sputum of patients with diseases, for example, caused by β -lactamase producing bacteria, such as urethral infection and pneumonia, and nosocomial infections derived from *Klebsiella pneumoniae* and *Escherichia coli*.

### Examples

### 1. Preparation of solution containing reagent composition for detecting β -lactamase

1.25 mg of a β -lactamase detection substrate and 1.25 mg of a β -lactamase inhibitor, both of which are shown in the following Table 1, were dissolved in a mixed solvent of 0.1 mL of dimethyl sulfoxide and 0.9 mL of a phosphate buffer solution, to prepare a solution containing reagent composition for detecting β -lactamase.

**Table 1**

| Reagent No. | β -lactamase detection substrate | β -lactamase inhibitor |
|---|---|---|
| 1 | Nitrocefin | -- |
| 2 | Nitrocefin | AZT, EDTA |
| 3 | Nitrocefin | CVA, EDTA |
| 4 | Nitrocefin | AZT, CVA |
| 5 | HMRZ compound | AZT, EDTA |
| 6 | HMRZ compound | AZT, CVA, EDTA |
| 7 | HMRZ compound | -- |
| 8 | Nitrocefin | AZT |
| 9 | Nitrocefin | CVA |
| 10 | Nitrocefin | AZT, CVA |
| 11 | HMRZ compound | AZT |
| 12 | HMRZ compound | AZT, CVA |

In the above table,
nitrocefin denotes 3-[2,4-dinitrostyryl]-7-(2-thienylacetamido]-3-cephem-4-carboxylic acid,
HMRZ compound denotes 7-[2-(2-aminothiazol-4-yl)-2-(1-carboxy-1-methylethoxy-imino)acetamido]-3-(2,4-dinitrostyryl)-3-cephem-4-carboxylic acid,
AZT denotes aztreonam, EDTA denotes ethylenediaminetetraacetic acid, and CVA denotes clavulanic acid.

### 2. Preparation of plate

The obtained solution containing reagent composition for detecting β -lactamase was applied to a microplate so that the amounts of the β -lactamase detection substrate and the β -lactamase inhibitor might individually be 12.5 µg per well.

### 3. Preparation of liquid specimen containing target substance to be analyzed

Using β -lactamase nonproducing bacteria, class-A β -lactamase- producing bacteria, class-B β -lactamase-producing bacteria, class-C β -lactamase-producing bacteria, and ESBL-producing bacteria, shown in the following Table 2, each of liquid specimens was prepared by inoculating each kind of bacteria into a phosphate buffer solution to have a concentration of 10⁸ CFU/mL.

**Table 2**

| Produced Enzyme | Name of Bacteria |
|---|---|
| Enzyme non-produced | *S*. *aureus* |
| Class A | *K. pneumoniae* |
| Class B | *S*. *marcescens* |
| Class C | *S*. *marcescens* |
| ESBL | *E*. *coli* |

### 4. Reactivities to β -lactamases

The liquid specimen was inoculated into the microplate prepared in the above in an amount of 50 µ L/well. The reactivities to the β-lactamases were examined by observing a change in color tone 30 minutes after inoculation.

**Table 3 Reactivities to β -lactamases**

| Produced Enzyme | Reagent No. | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Non-produced | - | - | - | - | - | - | - | - | - | - | - | - |
| Class A | + | + | - | - | - | - | - | + | - | - | - | - |
| Class B | + | - | - | + | - | - | ± | + | + | + | ± | ± |
| Class C | + | - | + | - | - | - | ± | - | + | - | - | - |
| ESBL | + | + | - | - | + | - | ± | + | - | - | + | - |

| | | | | | | | | | | | | |
|---|---|---|---|---|---|---|---|---|---|---|---|---|
| +: positive (The color changed from yellow to red.) negative (The yellow color was not changed.) ±: (A slight change was observed.) | | | | | | | | | | | | |

According to the present invention, β -lactamases can be detected rapidly and easily with high sensitivity. In addition, the combined use of a particular detecting reagent composition and a particular inhibitor can make it possible to detect even the class of β -lactamases, with a slight detection error in the present invention. Rapid discrimination of the class can lead to choice of a proper antibacterial agent, which provides a highly effective cure.

## Claims

1. A reagent composition for discriminating the class of β-lactamase for use with a chromogenic cephalosporin method, comprising a β-lactamase detection substrate and a β-lactamase inhibitor, wherein the β-lactamase inhibitor is selected from the group consisting of a combination of aztreonam and ethylendiaminetetraacetic acid; a combination of clavulanic acid and ethylendiaminetetraacetic acid; a combination of aztreonam and clavulanic acid.

2. The reagent composition for discriminating the class of β-lactamase as claimed in Claim 1, wherein the β-lactamase detection substrate is a compound represented by general formula (1) or physiologically acceptable salt thereof, or a compound represented by general formula (2) or carboxylate derivative thereof: 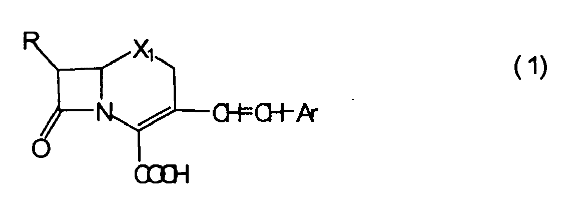 wherein R is a formamide group or a group represented by formula of R^{u}CH₂CONH, R^{u}OCH₂CONH, R^{u}CONH, R^{u}CH(NH₂)CONH, or R^{u}C(=NOH)CONH, wherein R^{u} is a thienyl group, phenyl group or naphthyl group; Ar is a phenyl group having a substitution of cyano group or nitro group at the 2-, 4-, or 2,4-position; and -X₁- is -S- or -SO-; 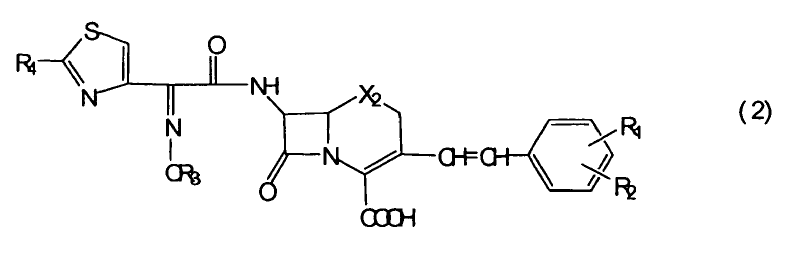 wherein R₁ and R₂, which may be the same or different, are each hydrogen atom, nitro group, or cyano group; R₃ is an alkyl group having 1 to 6 carbon atoms which may have a substituent of carboxyl group; R₄ is hydrogen atom or amino group; and -X₂- is -S- or -SO-, provided that R₁ and R₂ do not represent hydrogen atom at the same time.

3. A kit for discriminating the class of β-lactamase comprising one or more detecting reagent compositions selected from the group consisting of:
(a) a detecting reagent composition comprising as a β-lactamase detection substrate the compound represented by general formula (1) or physiologically acceptable salt thereof as claimed in Claim 2, and as a β-lactamase inhibitor a combination of aztreonam and ethylenediaminetetraacetic acid;
(b) a detecting reagent composition comprising as a β-lactamase detection substrate the compound represented by general formula (1) or physiologically acceptable salt thereof as claimed in Claim 2, and as a β-lactamase inhibitor a combination of clavulanic acid and ethylenediaminetetraacetic acid;
(c) a detecting reagent composition comprising as a β-lactamase detection substrate the compound represented by general formula (1) or physiologically acceptable salt thereof as claimed in Claim 2, and as a β-lactamase inhibitor a combination of aztreonam and clavulanic acid;
(d) a detecting reagent composition comprising as a β-lactamase detection substrate the compound represented by general formula (2) or carboxylate derivative thereof as claimed in Claim 2, and as a β-lactamase inhibitor a combination of aztreonam and ethylenediaminetetraacetic acid.

4. The kit for discriminating the class of β-lactamase as claimed in Claim 3, comprising as an essential composition the detecting reagent composition (c) as claimed in Claim 3, and further comprising one or more detecting reagent compositions selected from the group consisting of the detecting reagent compositions (a), (b), and (d) as claimed in Claim 3.

5. The kit for discriminating the class of β-lactamase as claimed in Claim 3, comprising as an essential composition the detecting reagent composition (d) as claimed in Claim 3, and further comprising one or more detecting reagent compositions selected from the group consisting of the detecting reagent compositions (a), (b), and (c) as claimed in Claim 3.

6. The kit for discriminating the class of β -lactamase as claimed in Claim 3, comprising all the detecting reagent compositions (a) through (d) as claimed in Claim 3.

7. A kit for discriminating the class of β -lactamase comprising one or more detecting reagent compositions selected from the group consisting of:
(h) a detecting reagent composition comprising as a β -lactamase detection substrate the compound represented by general formula (2) or carboxylate derivative thereof as claimed in Claim 2, and as a β -lactamase inhibitor aztreonam; and
(i) a detecting reagent composition comprising as a β -lactamase detection substrate the compound represented by general formula (2) or carboxylate derivative thereof as claimed in Claim 2, and as a β -lactamase inhibitor a combination of aztreonam and clavulanic acid.

8. The kit for discriminating the class of β -lactamase as claimed in Claim 7, further comprising the detecting reagent composition (c) as claimed in Claim 3.

9. The kit for discriminating the class of β -lactamase as claimed in Claim 7, comprising as an essential composition the detecting reagent composition (h) as claimed in Claim 7, and further comprising one or more detecting reagent compositions selected from the group consisting of
(f) a detecting reagent composition comprising as a β-lactamase detection substrate the compound represented by general formula (1) or physiologically acceptable salt thereof as claimed in Claim 2, and as a β-lactamase inhibitor aztreonam; and
(g) a detecting reagent composition comprising as a β-lactamase detection substrate the compound represented by general formula (1) or physiologically acceptable salt thereof as claimed in Claim 2, and as a β-lactamase inhibitor clavulanic acid; and
(i) a detecting reagent composition comprising as a β-lactamase detection substrate the compound represented by general formula (2) or carboxylate derivative thereof as claimed in Claim 2, and as a β-lactamase inhibitor a combination of aztreonam and clavulanic acid.

10. The kit for discriminating the class of β-lactamase as claimed in Claim 7, comprising all the detecting reagent compositions (h) and (i) as claimed in Claim 7 and the detecting reagent compositions (f) and (g) as claimed in Claim 9.

11. A method for discriminating the class of β-lactamase, comprising:
bringing a liquid specimen containing a target substance the class of which is to be discriminated into contact with the reagent composition for detecting β-lactamase as claimed in any one of Claims 1 or 2.

12. A method for discriminating the class of β-lactamase as claimed in Claim 11, wherein the reagent composition is held in a sample holding portion of a kit for detecting β-lactamase.

13. A method for discriminating the class of β-lactamase comprising:
bringing a liquid specimen containing a target substance which is to be discriminated into contact with a reagent composition for detecting β-lactamase held in a sample holding portion of the kit for detecting β-lactamase as claimed in any one of Claims 3 to 10.

14. Use of a β-lactamase detection substrate in combination with a β-lactamase inhibitor selected from the group consisting of a combination of aztreonam and ethylendiaminetetraacetic acid; a combination of clavulanic acid and ethylendiaminetetraacetic acid; a combination of aztreonam and clavulanic acid for the production of a reagent composition for discriminating the class of β-lactamase for use with a chromogenic cephalosporin method.

15. Use of claim 14, wherein the β-lactamase detection substrate is a compound represented by general formula (1) or physiologically acceptable salt thereof, or a compound represented by general formula (2) or carboxylate derivative thereof: 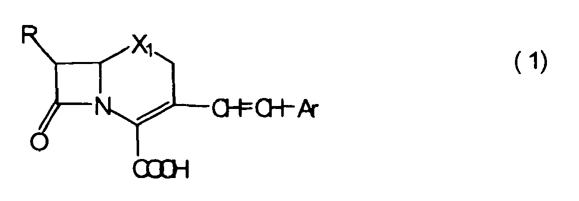 wherein R is a formamide group or a group represented by formula of R^{u}CH₂CONH, R^{u}OCH₂CONH, R^{u}CONH, R^{u}CH(NH₂)CONH, or R^{u}C(=NOH)CONH, wherein R^{u} is a thienyl group, phenyl group or naphthyl group; Ar is a phenyl group having a substitution of cyano group or nitro group at the 2-, 4-, or 2,4-position; and -X₁- is -S- or -SO-; 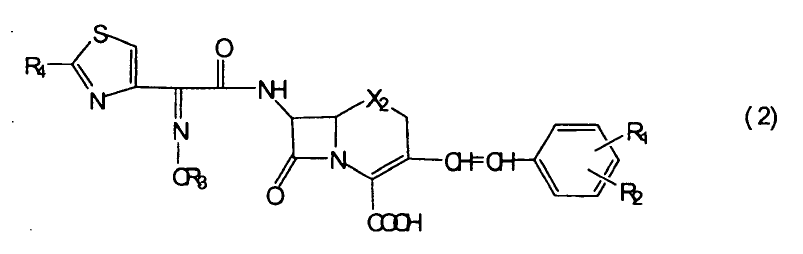 wherein R₁ and R₂, which may be the same or different, are each hydrogen atom, nitro group, or cyano group; R₃ is an alkyl group having 1 to 6 carbon atoms which may have a substituent of carboxyl group; R₄ is hydrogen atom or amino group; and -X₂- is -S- or -SO-, provided that R₁ and R₂ do not represent hydrogen atom at the same time.

## Patentansprüche

1. Reagenzzusammensetzung zur Diskriminierung der β-Lactamase-Klasse zur Verwendung mit einem chromogenen Cephalosporin-Verfahren, welche ein β-Lactamase-Detektionssubstrat und einen β-Lactamase-Inhibitor umfasst, wobei der β-Lactamase-Inhibitor ausgewählt ist aus der Gruppe bestehend aus einer Kombination von Aztreonam und Ethylendiamintetraessigsäure; einer Kombination von Clavulansäure und Ethylendiamintetraessigsäure; einer Kombination von Aztreonam und Clavulansäure.

2. Die Reagenzzusammensetzung zur Diskriminierung der β-Lactamase-Klasse gemäß Anspruch 1, wobei das β-Lactamase-Detektionssubstrat eine durch die allgemeine Formel (1) dargestellte Verbindung, oder ein physiologisch akzeptables Salz davon, oder eine durch die allgemeine Formel (2) dargestellte Verbindung oder ein Carboxylatderivat davon ist: 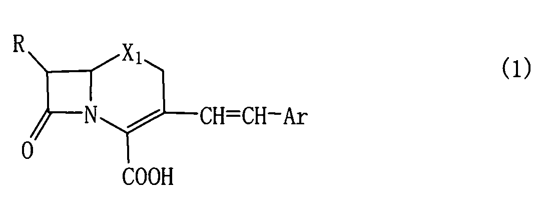 wobei R eine Formamidgruppe oder eine durch die Formel R^{u}CH₂CONH, R^{u}OCH₂CONH, R^{u}CONH, R^{u}CH(NH₂)CONH, oder R^{u}C(=NOH)CONH dargestellte Gruppe ist, wobei R^{u} eine Thienylgruppe, Phenylgruppe oder Naphthylgruppe ist; Ar eine Phenylgruppe ist, die mit einer Cyanogruppe oder einer Nitrogruppe an der 2-, 4-, oder 2,4-Position substituiert ist; und -X₁- -S- oder -SO- ist; 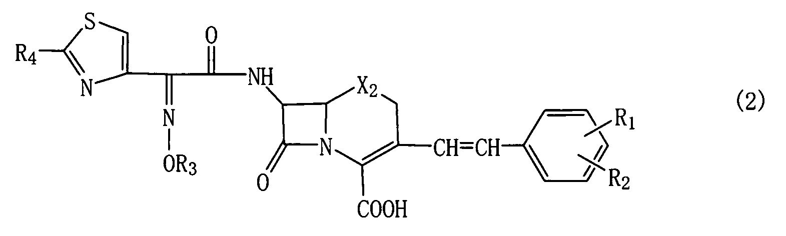 wobei R₁ und R₂, welche gleich oder unterschiedlich sein können, jeweils ein Wasserstoffatom, eine Nitrogruppe oder eine Cyanogruppe sind; R₃ eine Alkylgruppe mit 1 bis 6 Kohlenstoffatomen ist, welche einen Carboxylgruppe-Substituenten haben kann; R₄ ein Wasserstoffatom oder eine Aminogruppe ist; und -X₂- -S- oder -SO- ist, vorausgesetzt, dass R₁ und R₂ nicht zur gleichen Zeit Wasserstoffatome sind.

3. Kit zur Diskriminierung der β-Lactamase-Klasse, welcher eine oder mehrere Detektionsreagenzzusammensetzung(en) umfasst, welche ausgewählt sind aus der Gruppe bestehend aus:
(a) einer Detektionsreagenzzusammensetzung, welche als ein β-Lactamase-Detektionssubstrat, die durch die allgemeine Formel (1) dargestellte Verbindung oder ein physiologisch akzeptables Salz davon wie in Anspruch 2 beansprucht, umfasst, und welche als einen β-Lactamase-Inhibitor eine Kombination aus Aztreonam und Ethylendiamintetraessigsäure umfasst;
(b) einer Detektionsreagenzzusammensetzung, welche als ein β-Lactamase-Detektionssubstrat, die durch die allgemeine Formel (1) dargestellte Verbindung oder ein physiologisch akzeptables Salz davon wie in Anspruch 2 beansprucht, umfasst, und welche als einen β-Lactamase-Inhibitor eine Kombination aus Clavulansäure und Ethylendiamintetraessigsäure umfasst;
(c) einer Detektionsreagenzzusammensetzung, welche als ein β-Lactamase-Detektionssubstrat, die durch die allgemeine Formel (1) dargestellte Verbindung oder ein physiologisch akzeptables Salz davon wie in Anspruch 2 beansprucht, umfasst, und welche als einen β-Lactamase-Inhibitor eine Kombination aus Aztreonam und Clavulansäure umfasst;
(d) einer Detektionsreagenzzusammensetzung, welche als ein β-Lactamase-Detektionssubstrat, die durch die allgemeine Formel (2) dargestellte Verbindung oder ein Carboxylatderivat davon wie in Anspruch 2 beansprucht, umfasst, und welche als einen β-Lactamase-Inhibitor eine Kombination aus Aztreonam und Ethylendiamintetraessigsäure umfasst;

4. Kit zur Diskriminierung der β-Lactamase-Klasse gemäß Anspruch 3, welcher als eine wesentliche Zusammensetzung die Detektionsreagenzzusammensetzung (c) wie in Anspruch 3 beansprucht umfasst, und welcher des Weiteren eine oder mehrere Detektionsreagenzzusammensetzung(en) umfasst, welche ausgewählt sind aus der Gruppe bestehend aus den Detektionsreagenzzusammensetzungen (a), (b) und (d) wie in Anspruch 3 beansprucht.

5. Kit zur Diskriminierung der β-Lactamase-Klasse gemäß Anspruch 3, welcher als eine wesentliche Zusammensetzung die Detektionsreagenzzusammensetzung (d) wie in Anspruch 3 beansprucht umfasst, und welcher des Weiteren eine oder mehrere Detektionsreagenzzusammensetzung(en) umfasst, welche ausgewählt sind aus der Gruppe bestehend aus den Detektionsreagenzzusammensetzungen (a), (b) und (c) wie in Anspruch 3 beansprucht.

6. Kit zur Diskriminierung der β-Lactamase-Klasse gemäß Anspruch 3, welcher alle Detektionsreagenzzusammensetzungen (a) bis (d) wie in Anspruch 3 beansprucht, umfasst.

7. Kit zur Diskriminierung der β-Lactamase-Klasse, welcher eine oder mehrere Detektionsreagenzzusammensetzung(en) umfasst, welche ausgewählt sind aus der Gruppe bestehend aus:
h) einer Detektionsreagenzzusammensetzung, welche als ein β-Lactamase-Detektionssubstrat, die durch die allgemeine Formel (2) dargestellte Verbindung oder ein Carboxylatderivat davon wie in Anspruch 2 beansprucht, umfasst, und welche als einen β-Lactamase-Inhibitor Aztreonam umfasst; und
i) einer Detektionsreagenzzusammensetzung, welche als ein β-Lactamase-Detektionssubstrat, die durch die allgemeine Formel (2) dargestellte Verbindung oder ein Carboxylatderivat davon wie in Anspruch 2 beansprucht, umfasst, und welche als einen β-Lactamase-Inhibitor eine Kombination aus Aztreonam und Clavulansäure umfasst.

8. Kit zur Diskriminierung der β-Lactamase-Klasse gemäß Anspruch 7, welcher des Weiteren die Detektionsreagenzzusammensetzung (c), wie in Anspruch 3 beansprucht, umfasst.

9. Kit zur Diskriminierung der β-Lactamase-Klasse gemäß Anspruch 7, welcher als eine wesentliche Zusammensetzung die Detektionsreagenzzusammensetzung (h), wie in Anspruch 7 beansprucht, umfasst, und welcher des Weiteren eine oder mehrere Detektionsreagenzzusammensetzungen umfasst, welche ausgewählt sind aus der Gruppe bestehend aus:
(f) einer Detektionsreagenzzusammensetzung, welche als ein β-Lactamase-Detektionssubstrat die durch die allgemeine Formel (1) dargestellte Verbindung oder ein physiologisch akzeptables Salz davon, wie in Anspruch 2 beansprucht, umfasst, und welche als einen β-Lactamase-Inhibitor Aztreonam umfasst; und
(g) einer Detektionsreagenzzusammensetzung, welche als ein β-Lactamase-Detektionssubstrat die durch die allgemeine Formel (1) dargestellte Verbindung oder ein physiologisch akzeptables Salz davon, wie in Anspruch 2 beansprucht, umfasst, und als einen β-Lactamase-Inhibitor Clavulansäure umfasst; und
(i) einer Detektionsreagenzzusammensetzung, welche als ein β-Lactamase-Detektionssubstrat die durch die allgemeine Formel (2) dargestellte Verbindung oder ein Carboxylatderivat davon, wie in Anspruch 2 beansprucht, umfasst, und welche als einen β-Lactamase-Inhibitor eine Kombination aus Aztreonam und Clavulansäure umfasst.

10. Kit zur Diskriminierung der β-Lactamase-Klasse gemäß Anspruch 7, welcher alle Detektionsreagenzzusammensetzungen (h) und (i), wie in Anspruch 7 beansprucht, und die Detektionsreagenzzusammensetzungen (f) und (g), wie in Anspruch 9 beansprucht, umfasst.

11. Verfahren zur Diskriminierung der β-Lactamase-Klasse, welches umfasst: In-Kontakt-Bringen einer flüssigen Probe, welche eine Zielsubstanz deren Klasse zu diskriminieren ist, enthält mit der Reagenzzusammensetzung zur β-Lactamase-Detektion wie in einem der Ansprüche 1 oder 2 beansprucht.

12. Verfahren zur Diskriminierung der β-Lactamase-Klasse gemäß Anspruch 11, wobei die Reagenzzusammensetzung in einem Probenhalteteil des Kits zur β-Lactamase Detektion gehalten wird.

13. Verfahren zur Diskriminierung der β-Lactamase-Klasse, welches umfasst: In-Kontakt-Bringen einer flüssigen Probe, welche eine zu diskriminierende Zielsubstanz enthält, mit einer Reagenzzusammensetzung zur β-Lactamase-Detektion, welche in einer Probenhalteportion des Kits zur β-Lactamase-Detektion gehalten wird, wie in einem der Ansprüche 3 bis 10 beansprucht.

14. Verwendung eines β-Lactamase Detektionssubstrats in Kombination mit einem β-Lactamase-Inhibitor, welcher ausgewählt ist aus der Gruppe bestehend aus: einer Kombination von Aztreonam und Ethylendiamintetraessigsäure; einer Kombination von Clavulansäure und Ethylendiamintetraessigsäure; einer Kombination von Aztreonam und Clavulansäure zur Herstellung einer Reagenzzusammensetzung zur Diskriminierung der β-Lactamase-Klasse zur Verwendung mit einem chromogenen Cephalosporin-Verfahren.

15. Verwendung gemäß Anspruch 14, wobei das β-Lactamase-Detektionssubstrat eine durch die allgemeine Formel (1) dargestellte Verbindung, oder ein physiologisch akzeptables Salz davon, oder eine durch die allgemeine Formel (2) dargestellte Verbindung oder ein Carboxylatderivat davon ist: 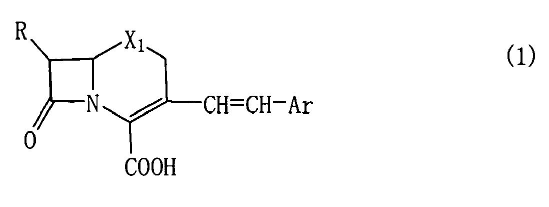 wobei R eine Formamidgruppe oder eine durch die Formel R^{u}CH₂CONH, R^{u}OCH₂CONH, R^{u}CONH, R^{u}CH(NH₂)CONH, oder R^{u}C(=NOH)CONH dargestellte Gruppe ist, wobei R^{u} eine Thienylgruppe, Phenylgruppe oder Naphthylgruppe ist; Ar eine Phenylgruppe ist, die mit einer Cyanogruppe oder einer Nitrogruppe an der 2-, 4-, oder 2,4-Position substituiert ist; und -X₁- -S- oder -SO- ist; 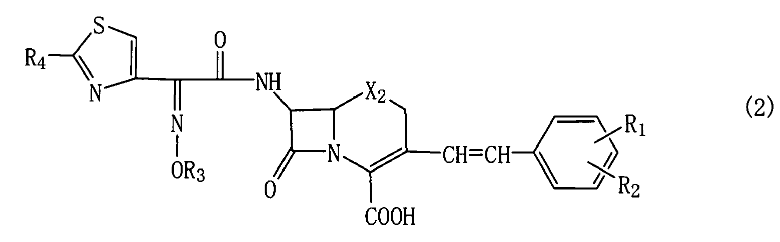 wobei R₁ und R₂, welche gleich oder unterschiedlich sein können, jeweils ein Wasserstoffatom, eine Nitrogruppe oder eine Cyanogruppe sind; R₃ eine Alkylgruppe mit 1 bis 6 Kohlenstoffatomen ist, welche einen Carboxylgruppen-Substituenten haben kann; R₄ ein Wasserstoffatom oder eine Aminogruppe ist; und -X₂- -S- oder - SO- ist, vorausgesetzt, dass R₁ und R₂ nicht zur gleichen Zeit Wasserstoffatome sind.

## Revendications

1. Composition réactive pour établir une discrimination dans la classe des β-lactamases à utiliser avec un procédé chromogène à base de céphalosporine, comprenant un substrat de détection de β-lactamase et un inhibiteur de β-lactamase, dans laquelle l'inhibiteur de β-lactamase est choisi dans le groupe constitué d'une combinaison d'aztréoname et d'acide éthylènediaminetétraacétique ; d'une combinaison d'acide clavulanique et d'acide éthylènediamine-tétraacétique ; et d'une combinaison d'aztréoname et d'acide clavulanique.

2. Composition réactive pour établir une discrimination dans la classe des β-lactamases selon la revendication 1, dans laquelle le substrat de détection de β-lactamase est un composé représenté par la formule générale (1) ou son sel physiologiquement acceptable ou un composé représenté par la formule générale (2) ou son dérivé carboxylate, 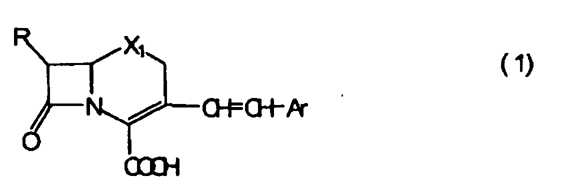 dans laquelle R est un groupement formamide ou un groupement représenté par les formules R^{u}CH₂CONH, R^{u}OCH₂CONH, R^{u}CONH, R^{u}CH (NH₂) CONH, ou R^{u}C (=NOH) CONH, où R^{u} est un groupement thiényle, phényle ou naphtyle ; Ar est un groupement phényle substitué par un groupement cyano ou nitro en position 2, 4 ou 2,4 ; et -X₁- représente -S- ou -SO- ; 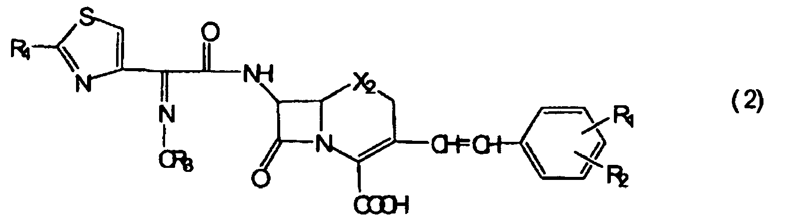 dans laquelle R₁ et R₂, qui peuvent être identiques ou différents, représentent respectivement un atome d'hydrogène, un groupement nitro ou un groupement cyano ; R₃ est un groupement alkyle ayant 1 à 6 atomes de carbone, qui peut avoir un substituant de groupement carboxyle ; R₄ est un atome d'hydrogène ou un groupement amino ; et -X₂- représente -S- ou -SO, à condition que R₁ et R₂ ne représentent pas en même temps un atome d'hydrogène.

3. Trousse pour établir une discrimination dans la classe des β-lactamases, comprenant une ou plusieurs compositions réactives de détection choisies dans le groupe constitué
(a) d'une composition réactive de détection comprenant comme substrat de détection de β-lactamase le composé représenté par la formule générale (1) ou son sel physiologiquement acceptable selon la revendication 2 et, comme inhibiteur de β-lactamase, une combinaison d'aztréoname et d'acide éthylènediaminetétraacétique ;
(b) d'une composition réactive de détection comprenant comme substrat de détection de β-lactamase le composé représenté par la formule générale (1) ou son sel physiologiquement acceptable selon la revendication 2 et, comme inhibiteur de β-lactamase, une combinaison d'aztréoname et d'acide éthylènediaminetétraacétique ;
(c) d'une composition réactive de détection comprenant comme substrat de détection de β-lactamase le composé représenté par la formule générale (1) ou son sel physiologiquement acceptable selon la revendication 2 et, comme inhibiteur de β-lactamase, une combinaison d'aztréoname et d'acide clavulanique ;
(d) d'une composition réactive de détection comprenant comme substrat de détection de β-lactamase le composé représenté par la formule générale (2) ou son dérivé carboxylate selon la revendication 2 et, comme inhibiteur de β-lactamase, une combinaison d'aztréoname et d'acide éthylène-diaminetétraacétique.

4. Trousse pour établir une discrimination dans la classe des β-lactamases selon la revendication 3, comprenant, comme composition essentielle, la composition réactive de détection (c) selon la revendication 3 et comprenant en outre une ou plusieurs compositions réactives de détection choisies dans le groupe constitué des compositions réactives de détection (a), (b) et (d) selon la revendication 3.

5. Trousse pour établir une discrimination dans la classe des β-lactamases selon la revendication 3, comprenant, comme composition essentielle, la composition réactive de détection (d) selon la revendication 3 et présentant en outre une ou plusieurs compositions réactives de détection choisies dans le groupe constitué des compositions réactives de détection (a), (b) et (c) selon la revendication 3.

6. Trousse pour établir une discrimination dans la classe des β-lactamase selon la revendication 3, comprenant toutes les compositions réactives de détection (a) à (d) selon la revendication 3.

7. Trousse pour établir une discrimination dans la classe des β-lactamases comprenant une ou plusieurs compositions réactives de détection choisies dans le groupe constitué :
(h) d'une composition réactive de détection comprenant comme substrat de détection de β-lactamase le composé représenté par la formule générale (2) ou son dérivé carboxylate selon la revendication 2 et comme inhibiteur de β-lactamase, l'aztréoname ; et
(i) d'une composition réactive de détection comprenant comme substrat de détection de β-lactamase le composé représenté par la formule générale (2) ou son dérivé carboxylate selon la revendication 2 et, comme inhibiteur de β-lactamase, une combinaison d'aztréoname et d'acide clavulanique.

8. Trousse pour établir une discrimination dans la classe des β-lactamases selon la revendication 7, comprenant en outre la composition réactive de détection (c) selon la revendication 3.

9. Trousse pour établir une discrimination dans la classe des β-lactamase selon la revendication 7, comprenant comme composition essentielle la composition réactive de détection (h) selon la revendication 7 et comprenant en outre une ou plusieurs compositions réactives de détection choisies dans le groupe constitué :
(f) d'une composition réactive de détection comprenant comme substrat de détection de β-lactamase le composé représenté par la formule générale (1) ou son sel physiologiquement acceptable selon la revendication 2 et, comme inhibiteur de β-lactamase, l'aztréoname ; et
(g) une composition réactive de détection comprenant comme substrat de détection de β-lactamase le composé représenté par la formule générale (1) ou son sel physiologiquement acceptable selon la revendication 2 et, comme inhibiteur de β-lactamase, l'acide clavulanique ; et
(i) une composition réactive de détection comprenant comme substrat de détection de β-lactamase le composé représenté par la formule générale (2) ou son dérivé carboxylate selon la revendication 2 et, comme inhibiteur de β-lactamase, une combinaison d'aztréoname et d'acide clavulanique.

10. Trousse pour établir une discrimination dans la classe des β-lactamases selon la revendication 7, comprenant toutes les compositions réactives de détection (h) et (i) selon la revendication 7 et les compositions réactives de détection (f) et (g) selon la revendication 9.

11. Procédé pour établir une discrimination dans la classe des β-lactamases, comprenant les étapes consistant à amener un échantillon de liquide contenant une substance cible dont la classe est à discriminer en contact avec la composition réactive pour détecter de la β-lactamase selon l'une quelconque des revendications 1 ou 2.

12. Procédé pour établir une discrimination dans la classe des β-lactamases selon la revendication 11, dans lequel la composition réactive est conservée dans une partie de conservation d'échantillon d'une trousse de détection de β-lactamase.

13. Procédé pour établir une discrimination dans la classe des β-lactamase comprenant les étapes consistant à amener un échantillon de liquide contenant une substance cible dont la classe est à discriminer en contact avec la composition réactive pour détecter de la β-lactamase conservée dans une partie de conservation d'échantillon d'une trousse de détection de β-lactamase selon l'une quelconque des revendications 3 à 10.

14. Utilisation d'un substrat de détection de β-lactamase en combinaison avec un inhibiteur de β-lactamase choisi dans le groupe constitué d'une combinaison d'aztréoname et d'acide éthylène-diaminetétraacétique ; d'une combinaison d'aztréoname et d'acide clavulanique pour la production d'une composition réactive pour établir une discrimination dans la classe des β-lactamase pour usage avec un procédé chromogène à base de céphalosporine.

15. Utilisation selon la revendication 14, dans laquelle le substrat de détection de β-lactamase est un composé représenté par la formule générale (1) ou son sel physiologiquement acceptable, ou un composé représenté par la formule générale (2) ou son dérivé carboxylate, 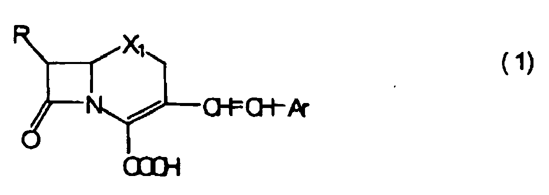 dans laquelle R est un groupement formamide ou un groupement représenté par les formules R^{u}CH₂CONH, R^{u}OCH₂CONH, R^{u}CONH, R^{u}CH(NH2)CONH, ou R^{u}C(=NOH)CONH, où R^{u} est un groupement thiényle, phényle ou naphtyle ; Ar est un groupement phényle substitué par un groupement cyano ou nitro en position 2, 4, ou 2, 4 ; et -X₁- représente -S- ou -SO- ; 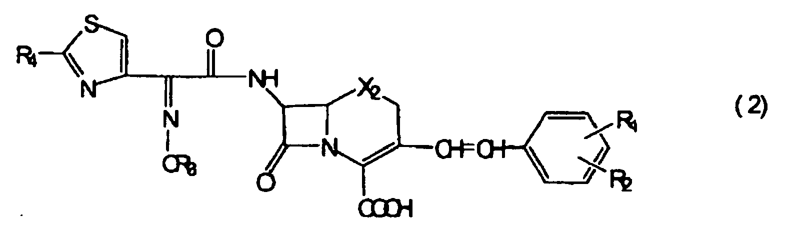 dans laquelle R₁ et R₂, qui peuvent être identiques ou différents, représentent respectivement un atome d'hydrogène, un groupement nitro ou un groupement cyano ; R₃ est un groupement alkyle ayant 1 à 6 atomes de carbone, qui peut avoir un substituant de groupement carboxyle ; R₄ est un atome d'hydrogène ou un groupement amino ; et -X₂- représente -S- ou -SO, à condition que R₁ et R₂ ne représentent pas en même temps un atome d'hydrogène.
